# EUROPEAN PATENT APPLICATION

(11) **EP 3 663 082 A1**
(43) Date of publication of application: **10.06.2020**
(21) Application number: 18842240.6
(22) Date of filing: 06.07.2018
(51) Int. Cl.: B32B 3/30, B05C 3/09, B05C 11/08, B05C 13/02, B05D 3/00, B05D 7/24, B32B 9/00

(54) **THREE-DIMENSIONAL STRUCTURE, METHOD FOR MANUFACTURING SAME, AND COATING DEVICE**

(30) Priority: 01.08.2017 JP 2017148854
(71) Applicant: Ishihara Sangyo Kaisha, Ltd., Osaka-shi, Osaka 550-0002 (JP)
(72) Inventor: KAJI, Seiji, Osaka-shi Osaka 550-0002 (JP)
(74) Representative: dompatent von Kreisler Selting Werner - Partnerschaft von Patent- und Rechtsanwälten mbB
(86) International application number: PCT/JP2018/025631
(87) International publication number: WO 2019/026539

(57) **Abstract**

Provided is a three-dimensional structure that makes it possible to obtain a coating film having a uniform thickness and good adhesion even when a three-dimensional structure main body has a concave section and/or a convex section, and that therefore has high durability without the coating film being peeled off even after long-term use. Also provided are a method for producing the three-dimensional structure, and a coating device that can be suitably used for this production method. The three-dimensional structure of the present invention has a three-dimensional structure main body having a concave section and/or a convex section on a surface and a coating film having a thickness of 10 nm to 300 nm and formed on a surface of the three-dimensional structure main body including the concave section and/or the convex section, wherein the coating film is made of a metal alkoxide or non-metal alkoxide hydrolysis product; and when a portion of the coating film located on a surface of the concave section and/or convex section in the three-dimensional structure main body is observed with a scanning electron microscope at a magnification of 300, no peeling of the coating film can be recognized.

## Description

### [Technical Field]

The present invention relates to a three-dimensional structure having a coating film includes a metal alkoxide or non-metal alkoxide hydrolysis product on the surface, a method for producing the same, and a coating device used in the method for producing the three-dimensional structure.

### [Background Art]

Three-dimensional structures in which a coating film include an oxide such as titanium oxide, silicon oxide, aluminum oxide, and zirconium oxide is formed on the surface of a substrate can be used in various applications such as catalysts, catalyst supports, adsorbents, photocatalysts, electrodes, artificial bones, and the like. For example, a three-dimensional structure formed with a coating film includes titanium oxide is used as a decomposition catalyst support for NOₓ or SOₓ, a photocatalyst for artificial photosynthesis, a solar cell electrode, a fuel cell catalyst electrode, a bone repair material, or the like.

A sol-gel method in which a substrate is immersed in a sol obtained by hydrolyzing a metal alkoxide or non-metal alkoxide and dried is generally used as a method for forming an oxide coating film on the surface of a substrate. For example, Patent Literature 1 to 3 and Non Patent Literature 1 disclose a method of forming a coating film includes titanium oxide by a general sol-gel method using titanium alkoxide on the surface of a substrate made of a polymer or the like. The technique described in Patent Literature 1 and Non Patent Literature 1 uses a method (referred to hereinbelow as "dip coating") in which after immersing the substrate in a sol prepared by partially hydrolyzing titanium tetraisopropoxide (TTIP), the substrate is pulled out of the sol, and then the substrate is dried (see Examples of Patent Literature 1).

A method (referred to hereinbelow as "spin coating") in which a substrate is fixed to a spin coater, a sol or the like composed of a titanium tetraisopropoxide partial hydrolysis product is dropped and coated on the substrate while rotating the substrate, and the coating is thereafter dried is also known as another method for forming an oxide coating film on the surface of a substrate (see Patent Literature 4).

### [Citation List]

### [Patent Literature]

[Patent Literature 1] Japanese Patent Application Publication No. 2015-136553
[Patent Literature 2] Publication of Japanese Patent No. 4606165
[Patent Literature 3] Publication of Japanese Patent No. 5271907
[Patent Literature 4] Publication of Japanese Patent No. 6073293

### [Non Patent Literature]

[Non Patent Literature 1] Takashi Kizuki, et al. Apatite-forming PEEK with TiO2 surface layer coating; J Mater Sci: Mater Med (2015) 26:41

### [Summary of Invention]

### [Technical Problem]

In the techniques described in Patent Literature 1 and Non Patent Literature 1, a coating film made of titanium oxide having good adhesion can be obtained on a flat region of a material by performing the dip coating on a disk-shaped substrate made of, for example, polyetheretherketone (PEEK) and then performing a drying.

However, where the above technique is applied to a substrate having a concave section or a convex section on the surface, there is a problem that the obtained coating film is easily peeled off at the peripheral edge portion of the concave section or the top portion of the convex section in the substrate. This is presumed to be due to the following reason.

When a dip coating is performed on a substrate having a concave section or a convex section on surface, the sol is likely to be stored at the bottom portion of the concave section or the base portion of the convex section on the substrate. Therefore, the coating film obtained after drying locally increases in thickness at the bottom portion of the concave section and the base portion of the convex section in the substrate, resulting in film thickness unevenness. As a result, a large stress is generated in the coating film, this stress causes fissure and cracks in a portion with a relatively large thickness, and peeling occurs.

Various problems occur depending on the use of the three-dimensional structure when the peeling occurs in the coating film as described above.

For example, an interbody spacer has a sawtooth-shaped concavo-convex shape on the surface in order to prevent a cage migration after being inserted in vivo. A problem associated with such a three-dimensional structure is that when the adhesion of the coating film includes titanium oxide on the surface is low, the coating film peels off from the substrate when used as a bone repair material or the like and no integration with the bone is achieved.

Meanwhile, it is possible to avoid peeling of the coating film by appropriately setting the conditions for the dip coating and forming a coating film with a thin thickness. However, when a coating film having a thin thickness is formed, the function of the coating film (for example, bone-bonding ability) is lost. Therefore, such an approach is not practical.

As described above, the techniques described in Patent Literature 1 and Non Patent Literature 1 are useful for a substrate having a flat surface on which a coating film is to be formed, but practical problems arise when the techniques are applied to three-dimensional structure having concavo-convex sections on the surface such as an interbody spacer.

Patent Literature 2 to 4 describe oxide coating methods using dip coating or spin coating, but all these methods are directed to a substrate having a flat surface. Therefore, it cannot be said that Patent Literature 2 to 4 clearly describe specific means as oxide coating methods applicable to a three-dimensional structure having concavo-convex sections on the surface such as an interbody spacer.

An object of the present invention is to provide a three-dimensional structure that makes it possible to obtain a coating film having a uniform thickness and good adhesion even when a three-dimensional structure main body has a concave section and/or a convex section, and that therefore has high durability without the coating film being peeled off even after long-term use, and also to provide a method for producing the same, and a coating device used in the method for producing the three-dimensional structure.

### [Solution to Problem]

As a result of intensive studies to solve the above problems, the present inventors have found that by coating the surface of a three-dimensional structure main body having a concave section and/or a convex section on the surface with a metal alkoxide or non-metal alkoxide partial hydrolysis product and also rotating the three-dimensional structure main body, the alkoxide partial hydrolysis product is coated with a uniform thickness, and as a result, a coating film having excellent adhesion is formed. The present invention has been accomplished based on this finding.

The three-dimensional structure of the present invention has a three-dimensional structure comprising:
a three-dimensional structure main body having a concave section and/or a convex section on a surface, and having a coating film on the surface that includes the concave section and/or convex section of the three-dimensional structure main body, and the coating film has a thickness of 10 nm to 300 nm, wherein
the coating film includes a metal alkoxide or non-metal alkoxide hydrolysis product; and
no cracks or peelings of the coating film can be recognized when a portion of the coating film located on the surface of the concave section and/or convex section in the three-dimensional structure main body is observed with a scanning electron microscope at a magnification of 300.

In the three-dimensional structure of the present invention, the coating film preferably has an adhesion strength measured by a 180 degree peeling test in accordance with JIS K 6854 of 40 N/10 mm or higher.

Further, the three-dimensional structure main body is preferably made of at least one material selected from the group consisting of polymers, metals and ceramics.

Further, the three-dimensional structure main body is preferably made of polyetheretherketone.

The coating film preferably includes a titanium alkoxide hydrolysis product.

The method for producing a three-dimensional structure of the present invention comprises a main body preparation step of preparing a three-dimensional structure main body having a concave section and/or a convex section on a surface;
a dispersion liquid preparation step of preparing a coating film precursor dispersion liquid including coating film precursor includes an alkoxide partial hydrolysis product by mixing a metal alkoxide or non-metal alkoxide with water;
a precursor coating step of coating the coating film precursor dispersion liquid on the surface of the three-dimensional structure main body; and
a coating film forming step of forming a coating film includes the alkoxide hydrolysis product on the surface of the three-dimensional structure main body by rotating the three-dimensional structure main body which has been coated with the coating film precursor dispersion liquid so that a centrifugal force acts on a center of gravity thereof.

It is preferable that the method for producing a three-dimensional structure of the present invention includes a main body modification treatment step of modifying the surface of the three-dimensional structure main body before performing the precursor coating step.

Further, it is preferable to form a coating film includes an alkoxide hydrolysis product is formed by rotating the three-dimensional structure main body while coating the coating film precursor dispersion liquid on the surface of the three-dimensional structure main body.

Further, in the method for producing a three-dimensional structure of the present invention, it is preferable that the three-dimensional structure main body is fixed to a rotating plate, and
the three-dimensional structure main body is immersed in the coating film precursor dispersion liquid and then separated from the coating film precursor dispersion liquid, thereby coating the coating film precursor dispersion liquid on the surface of the three-dimensional structure main body, and the three-dimensional structure main body be thereafter rotated by rotating the rotating plate to form a coating film includes the alkoxide hydrolysis product.

In such a method for producing a three-dimensional structure, it is preferable that the three-dimensional structure main body is fixed so that the whole of the three-dimensional structure main body is spaced apart from the rotation axis of the rotating plate.

Further, in the coating film forming step, it is preferable that the three-dimensional structure main body is autorotated in the direction opposite to the rotation direction of the rotating plate while rotating the three-dimensional structure main body.

In the method for producing a three-dimensional structure of the present invention, in the dispersion liquid preparation step, it is preferable that the coating precursor dispersion liquid is prepared by mixing 37 parts by mole of an organic solvent, 0.08 parts by mole to 1.5 parts by mole of a metal alkoxide or non-metal alkoxide, and water.

The alkoxide is preferably a titanium alkoxide.

In the coating film forming step, it is preferable that the relative centrifugal acceleration of the centrifugal force acting on the center of gravity of the three-dimensional structure main body is 10 G to 500 G.

In the coating film forming step, it is preferable that the three-dimensional structure main body is rotated and then subjected to drying at a temperature of 50°C to 200°C.

A coating device of the present invention is for coating a metal alkoxide or non-metal alkoxide partial hydrolysis product and/or alkoxide hydrolysis product on a surface of a three-dimensional structure main body having a concave section and/or a convex section on the surface, the coating device comprising:
coating means for coating a dispersion liquid including the alkoxide partial hydrolysis product on the surface of the three-dimensional structure main body; and
rotating means for rotating the three-dimensional structure main body so that a centrifugal force acts on a center of gravity thereof.

Moreover, a coating device of the present invention is for coating a metal alkoxide or non-metal alkoxide partial hydrolysis product and/or alkoxide hydrolysis product on the surface of a three-dimensional structure main body having a concave section and/or a convex section on the surface, the coating device comprising:
a rotating plate for fixing the three-dimensional structure main body;
coating means for coating a dispersion liquid including the alkoxide partial hydrolysis product on a surface of the three-dimensional structure main body fixed to the rotating plate; and
rotating means for rotating the three-dimensional structure main body fixed to the rotating plate, so that a centrifugal force acts on a center of gravity of the three-dimensional structure main body.

In the coating device of the present invention, it is preferable that the coating means is based on immersing the three-dimensional structure main body in the dispersion liquid.

Further, it is preferable that the coating means is based on immersing the three-dimensional structure main body in the dispersion liquid and then separating the three-dimensional structure main body from the dispersion liquid.

Further, in the coating device having the rotating plate, it is preferable that the fixing position of the three-dimensional structure main body on the rotating plate be set apart from a rotation axis of the rotating plate.

Moreover, it is preferable that autorotating means be provided for autorotating the three-dimensional structure main body in the direction opposite to the rotation direction of the rotating plate.

Moreover, it is preferable that a plurality of the three-dimensional structure main bodies is fixed to the rotating plate.

Further, in the coating device of the present invention, it is preferable that the rotating means apply a centrifugal force having a centrifugal acceleration of 10 G to 500 G to the center of gravity of the three-dimensional structure main body.

Moreover, it is preferable that drying means is provided for drying the alkoxide partial hydrolysis product and/or alkoxide hydrolysis product coated on a surface of the three-dimensional structure main body.

### [Advantageous Effects of Invention]

With the three-dimensional structure of the present invention, a coating film having a uniform thickness and good adhesion can be obtained even when the three-dimensional structure main body has a concave section and/or a convex section. Therefore, the three-dimensional structure of the present invention has high durability without the coating film being peeled off even after long-term use.

Moreover, according to the method for producing a three-dimensional structure of the present invention, a coating film having a uniform thickness and good adhesion can be obtained even when the three-dimensional structure main body has a concave section and/or a convex section, and therefore, it is possible to produce a three-dimensional structure that has high durability without the coating film being peeled off even after long-term use.

Furthermore, with the coating device of the present invention, the alkoxide partial hydrolysis product and/or the alkoxide hydrolysis product can be coated with a uniform thickness on the surface of a three-dimensional structure main body which has a concave section and/or a convex section.

### [Brief Description of Drawings]

[Fig. 1] Fig. 1 is a photograph showing an example of a three-dimensional structure of the present invention.
[Fig. 2] Fig. 2 is an explanatory drawing showing the configuration in one example of the coating device of the present invention.
[Fig. 3] Fig. 3 is a planar view of the rotating plate in the coating device shown in Fig. 2.
[Fig. 4] Fig. 4 is an explanatory drawing showing the configuration of rotating means in the coating device shown in Fig. 2.
[Fig. 5] Fig. 5 is an explanatory drawing showing a three-dimensional structure main body used in the Examples.
[Fig. 6] Fig. 6 is an explanatory drawing showing a position where a three-dimensional structure is fixed on a rotating plate in the Examples.
[Fig. 7-1] Fig. 7-1 is an electron micrograph taken at a magnification of 50 of a concavo-convex section in the three-dimensional structure produced in Example 1.
[Fig. 7-2] Fig. 7-2 is an electron micrograph taken at a magnification of 300 of a concavo-convex section in the three-dimensional structure produced in Example 1.
[Fig. 7-3] Fig. 7-3 is an electron micrograph taken at a magnification of 1,000 of a concavo-convex section in the three-dimensional structure produced in Example 1.
[Fig. 8-1] Fig. 8-1 is an electron micrograph taken at a magnification of 50 of a concavo-convex section in the three-dimensional structure produced in Comparative Example 1.
[Fig. 8-2] Fig. 8-2 is an electron micrograph taken at a magnification of 300 of a concavo-convex section in the three-dimensional structure produced in Comparative Example 1.
[Fig. 8-3] Fig. 8-3 is an electron micrograph taken at a magnification of 1,000 of a concavo-convex section in the three-dimensional structure produced in Comparative Example 1.

### [Description of Embodiments]

Hereinafter, embodiments for carrying out the present invention will be described in detail.

### [Three-Dimensional Structure]

The three-dimensional structure of the present invention is obtained by forming a coating film includes a metal or non-metal alkoxide hydrolysis product on the surface of a three-dimensional structure main body having a concave section and/or a convex section on the surface.

### (1) Three-Dimensional Structure Main Body

The three-dimensional structure main body has a concave section and/or a convex section on the surface. The depth of the concave section or the height of the convex section is in the range of 0.2 mm or higher, preferably in the range of 0.2 mm to 20 mm, and more preferably in the range of 0.2 mm to 5 mm. Further, the width of the concave section or the convex section is, for example, 2.0 mm to 15 mm.

The three-dimensional structure main body needs to have at least one concave section and/or convex section on the surface depending on the use of the three-dimensional structure, such as a bone repair material, a bone defect prosthetic material, and the like. For example, the three-dimensional structure main body having a concave section and/or a convex section is inclusive of a three-dimensional structure main body having at least a pair of concavo-convex structures on the surface, and a configuration having a surface in contact with the outer space inside thereof.

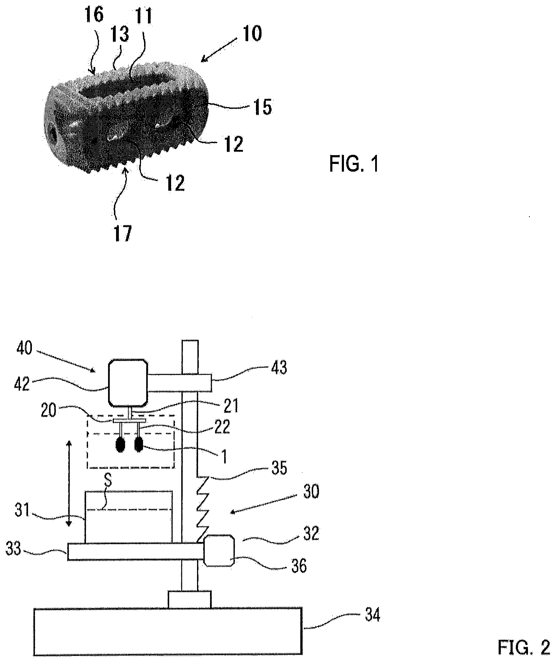

Examples of the three-dimensional structure main body having such a structure include a structure used as a cage having a cavity inside thereof, specifically, a structure used as a interbody cage 10 having the configuration shown in the photograph in Fig. 1. The interbody cage 10 shown in Fig. 1 has a substantially rectangular parallelepiped shape extending in the front-rear direction, and has a through-hole 11 having a rectangular cross section and penetrating from an upper surface portion 16 to a lower surface portion 17. A plurality of lateral holes 12 connecting with the through-hole 11 is formed in a side surface portion 15 of the interbody cage 10.

Further, a plurality of ridges 13 extending in a direction perpendicular to the surface of the side surface portion 15 is formed in parallel to each other on the surfaces of the upper surface portion 16 and the lower surface portion 17 of the interbody cage 10. The plurality of ridges 13 form concave sections and convex sections of the three-dimensional structure main body.

As the material of the three-dimensional structure main body, at least one selected from the group consisting of polymers, metals, and ceramics (including glass) can be used.

The specific materials of the three-dimensional structure main body will be described by taking as an example the case where the three-dimensional structure is used as a bone repair material or the like. Examples of suitable polymers include polyacrylic acid, polymethacrylic acid and these salts thereof, polyethylene, polypropylene, polytetrafluoroethylene, tetrafluoroethylene - perfluoroalkyl vinyl ether copolymer, tetrafluoroethylene - hexafluoropropylene copolymer, tetrafluoroethylene - ethylene copolymer, polyvinylidene fluoride, polychlorotrifluoroethylene, chlorotrifluoroethylene - ethylene copolymer, polyethylene terephthalate, polyamides, polyurethanes, polysiloxanes, polysiloxane elastomers, polyarylketone resins, polysulfone resins, and the like.

The polyarylketone resin is a thermoplastic resin having an aromatic nucleus bond, an ether bond and a ketone bond in a structural unit thereof, and many such resins have a linear polymer structure in which benzene rings are bonded by an ether bond and a ketone bond. Representative examples of the polyarylketone resin include polyetherketone (PEK), polyetheretherketone (PEEK), polyetherketoneketone (PEKK), polyetherketoneetherketoneketone (PEKEKK), and the like. Among these, from the viewpoint of having an elastic modulus close to that of bones, it is preferable that polyetheretherketone (PEEK) is used as the polymer constituting the three-dimensional structure main body.

The polysulfone resin (PSF) is an amorphous thermoplastic resin having a sulfonyl group in a structural unit thereof, and many such resins include an aromatic ring for high functionality. A polyethersulfone resin (PES) and a polyphenylsulfone resin (PPSU) are also included as sulfonyl group-containing resins in the polysulfone resins.

Further, various metals that are generally suitable for bone repair materials can be used. Specific examples of such metals include titanium, zirconium, hafnium, vanadium, niobium, tantalum, cobalt, iridium, and alloys thereof. Among these, titanium or a titanium alloy is preferable.

Further, various ceramics that are generally suitable for bone repair materials can be used. Specific examples of such ceramics include titanium dioxide, zirconium oxide, hafnium oxide, vanadium oxide, niobium oxide, tantalum oxide, cobalt oxide, iridium oxide. Examples of ceramics suitable for other applications include silicon oxide, aluminum oxide and cordierite.

The surface on which the coating film in the three-dimensional structure main body is to be formed is preferably subjected, as necessary, to a modification treatment. Specifically, the surface of the three-dimensional structure main body preferably has a water contact angle of 0° to 40°, more preferably 0° to 30°, and still more preferably 0° to 20°.

### (2) Coating Film

The coating film includes a metal alkoxide or non-metal alkoxide hydrolysis product.

The alkoxide hydrolysis product is obtained by drying-induced gelling of a sol formed by partial hydrolysis and polymerization of an alkoxide. Specifically, when a sol composed of an alkoxide partial hydrolysis product is dried, an alkoxide hydrolysis product is generated by the progress of the hydrolysis reaction and polymerization reaction, and this alkoxide hydrolysis product is called, in general, an oxide. Since the coating film is formed of a metal alkoxide or non-metal alkoxide hydrolysis product, it has a high light transmittance. When the light transmittance of the coating film is expressed as a haze (cloudiness), it is preferably 5 or less, more preferably 3 or less, and still more preferably 1 or less. The haze is measured based on the method described in JIS K 7136.

As the metal alkoxide for obtaining the alkoxide hydrolysis product, alkoxides of titanium, zirconium, hafnium, vanadium, niobium, tantalum, cobalt, iridium, aluminum and the like can be used.

Further, a silicon alkoxide can be used as the non-metal alkoxide for obtaining the alkoxide hydrolysis product.

Preferred alkoxides are a titanium alkoxide and a silicon alkoxide.

Examples of alcohols for obtaining the alkoxide include aliphatic alcohols having 1 to 16 carbon atoms such as ethanol, n-propanol, isopropanol, n-butanol, isobutanol, tert-butanol, 1-octanol, isooctyl alcohol, 2-ethylhexanol and the like. Of these, isopropanol, ethanol, n-propanol, and n-butanol are preferred.

The thickness of the coating film is 10 nm to 300 nm, preferably 20 nm to 200 nm, and more preferably 30 nm to 100 nm. When the thickness of the coating film is in the above range, the adhesion to the three-dimensional structure main body is good, and a coating film having a required function can be reliably obtained. When the thickness of the coating film is too thin, it may be difficult to obtain a coating film having a necessary function. Meanwhile, where the thickness of the coating film is too thick, the coating film may be cracked and peeled off from that portion.

The thickness of the coating film can be measured by observing a cross section using a transmission electron microscope. As another measurement method, optical measurement can be performed using an optical interference type film thickness meter (for example, "FE-3000", manufactured by Otsuka Electronics Co., Ltd.), an ellipsometer (for example, "UVISEL2", Horiba, Ltd.), and the like.
Further, a method (calibration curve method) of measuring the optical density of the sample obtained (UV absorptivity by regular transmitted light measured using "UV-2550" (Shimadzu Corporation)) and calculating the film thickness by using a calibration curve prepared in advance is also effective. In addition, it is also possible to prepare a sample for measuring the thickness of the coating film by coating artificially an alkoxide partial hydrolysis product, which is to be used for coating a three-dimensional structure, on a borosilicate glass substrate and rotating under the same conditions as the actual three-dimensional structure.

### (3) Characteristics of coating film

The coating film is such that when the portion of the coating film located on the surface of the concave section and/or convex section in the three-dimensional structure is observed with a scanning electron microscope at a magnification of 300, preferably at a magnification of 1,000, no peeling of the coating film can be recognized.

The coating film preferably has an adhesion strength measured by a 180 degree peeling test in accordance with JIS K 6854 of 40 N/10 mm or higher, more preferably 41 N/10 mm or higher, and still more preferably 43 N/10 mm or higher.

In the 180 degree peeling test, a peeling test tape (for example, "Y-4950" manufactured by 3M Co.) having a T-type peeling force (target SUS304) of 34 N/10 mm and a width of 10 mm is prepared. After affixing the peeling test tape to the surface of the coating film, the tape is pulled at a speed of 300 mm/min in the direction at 180 degrees, the peel strength is measured, and the measured value is taken as the adhesion strength.

Such a coating film is one in which peeling of the coating film is not observed when a transparent pressure-sensitive adhesive tape specified in JIS K 5600 is affixed and peeled off. This transparent pressure-sensitive adhesive tape has a width of 25 mm and an adhesion strength of 4 N/10 mm.
As the transparent pressure-sensitive adhesive tape, for example, "Cellotape (registered trademark)" manufactured by Nichiban Co., Ltd. can be used.

Where such conditions are satisfied, high durability can be obtained without the coating film being peeled off even after long-term use.

### (4) Application of Three-Dimensional Structure

With the three-dimensional structure of the present invention, a coating film having a uniform thickness and good adhesion can be obtained even when the three-dimensional structure main body has a concave section and/or a convex section. Therefore, the three-dimensional structure of the present invention has high durability without the coating film being peeled off even after long-term use.

Such a three-dimensional structure can be used in various applications, for example, a catalyst, a catalyst support, an adsorbent, a photocatalyst, an electrode, an artificial bone, and the like, depending on the material of the three-dimensional structure main body and the type of alkoxide hydrolysis product constituting the coating film.

In a specific example, by forming a coating film with a titanium alkoxide hydrolysis product, the three-dimensional structure can be used as a decomposition catalyst support for NOₓ or SOₓ, a photocatalyst and the like for artificial photosynthesis, a solar cell electrode, a fuel cell catalyst electrode, and the like. Further, it can be used as a bone repair material for bones or teeth, specifically as an artificial bone, a bone defect prosthesis material or a filling material. It is also suitable for vertebral body formation, vertebroplasty, femur bone formation, skull defect repair, and the like, and used for various cages such as interbody cages. Further, the three-dimensional structure is also used as a joint prosthesis material. The shape and structure of the three-dimensional structure and the concavo-convex shape of the surface can be designed, as appropriate, according to such a use. In addition to the alkoxide hydrolysis product, the coating film may include a radiopaque substance such as barium sulfate or zirconium oxide, and an antibacterial substance such as silver, copper, antibiotics and the like.

### [Method for Producing Three-Dimensional Structure]

The method for producing a three-dimensional structure of the present invention includes a main body preparation step of preparing a three-dimensional structure main body having a concave section and/or a convex section on a surface; a dispersion liquid preparation step of preparing a coating film precursor dispersion liquid comprises a coating film precursor includes an alkoxide partial hydrolysis product; and a coating film forming step of forming a coating film includes the alkoxide hydrolysis product by rotating the three-dimensional structure main body so that a centrifugal force acts on a center of gravity thereof.

In addition, it is preferable to perform, as necessary, a main body modification treatment step of modifying the surface of the three-dimensional structure main body before performing the precursor coating step.

Furthermore, when the three-dimensional structure to be produced is used as a biomaterial, a bioactivation treatment step of bioactivating the surface of the coating film can be performed, as necessary, after performing the coating film forming step.

### (1) Main Body Preparation Step

The method for producing the three-dimensional structure main body is not particularly limited. Depending on the material of the three-dimensional structure main body and the application of the three-dimensional structure, for example, a three-dimensional structure main body having a required form can be produced by molding a material for forming the three-dimensional structure main body which is made of a polymer, a metal, ceramics (including glass), and the like, or by cutting a lump made of the material.

When producing the three-dimensional structure main body by molding, the specific molding method is not particularly limited. For example, when a polymer is used as the material of the three-dimensional structure main body, a three-dimensional shaping method using a 3D printer or the like can be used.

The surface of the produced three-dimensional structure is preferably subjected to washing treatment with water, alcohol or the like.

### (2) Main Body Modification Treatment Step

The main body modification treatment step of modifying the surface of the three-dimensional structure main body is performed, as necessary, in consideration of the material of the three-dimensional structure main body, the application of the three-dimensional structure, and the like.

For example, when the three-dimensional structure main body is composed of a polymer such as polyetheretherketone (PEEK), it is preferable that a hydrophilic group is imparted to the portion exposed on the surface of the three-dimensional structure main body, because a coating film precursor includes an alkoxide partial hydrolysis product can be formed more firmly on the surface of the three-dimensional structure main body.

Methods disclosed in Patent Literature 1 or Non Patent Literature 1, specifically, methods of ultraviolet irradiation treatment and plasma treatment in an oxygen atmosphere, are preferably used as modification treatment methods. When plasma treatment is selected, a preferable treatment time is 30 sec or longer, and a more preferable treatment time is 5 min or longer. When the ultraviolet irradiation treatment is selected, a preferable treatment time is 5 min or longer, and a more preferable treatment time is 30 min or longer.

It is considered that a hydrophilic group such as an oxycarbonyl group (-O-C=O) or a carbonyl group (-C=O) is formed on the surface of the three-dimensional structure main body by subjecting the three-dimensional structure main body made of a polymer to ultraviolet irradiation treatment or plasma treatment in an oxygen atmosphere.

As a modification treatment method other than the plasma treatment and ultraviolet irradiation treatment, for example, blasting treatment or acid etching treatment may be used. By adjusting the surface roughness of the surface of the three-dimensional structure main body by these treatments, the surface of the three-dimensional structure main body can be modified.

### (3) Dispersion Liquid Preparation Step

In the dispersion liquid preparation step, the coating film precursor dispersion liquid is composed of a sol including a partial hydrolysis product of a metal alkoxide or non-metal alkoxide (hereinafter, also simply referred to as "partial hydrolysis product") and is prepared by mixing a metal alkoxide or non-metal alkoxide with water.

Specific examples of the metal alkoxides used for preparing the coating film precursor dispersion liquid include titanium alkoxides based on aliphatic alcohols having 1 to 8 carbon atoms such as tetraethoxytitanate, tetra(n-propoxy)titanate, tetra(isopropoxy)titanate, tetra(n-butoxy)titanate, tetra(isobutoxy)titanate, tetra(tert-butoxy)titanate, and the like.

Specific examples of the non-metal alkoxides used for preparing the coating film precursor dispersion liquid include silicon alkoxides based on aliphatic alcohols having 2 to 16 carbon atoms such as tetraethoxyorthosilicate, tetra(n-butoxy)orthosilicate, tetra(isobutoxy)orthosilicate, tetra(tert-butoxy)orthosilicate, and the like.

Mixing of the metal alkoxide or non-metal alkoxide with water is preferably performed in the presence of an acid such as nitric acid or hydrochloric acid or an alkali such as ammonia.

The hydrolysis rate of the alkoxide in the partial hydrolysis product can be set as appropriate, but is preferably 5% to 70%, more preferably 10% to 50% in terms of mole. Here, the hydrolysis rate of the alkoxide can be calculated from the ratio between the amount of water to be added and the amount of water to 100% hydrolyze the alkoxide.

In order to adjust the concentration of the partial hydrolysis product in the coating film precursor dispersion liquid, it is preferable to dilute the alkoxide with an organic solvent such as alcohol and then hydrolyzed. Specifically, by mixing 37 parts by mole of an organic solvent, 0.08 parts by mole to 1.5 parts by mole and more preferably 0.09 parts by mole to 1.0 parts by mole of the alkoxide, and water, it is possible to prepare a coating film precursor dispersion liquid including, in a suitable concentration, a coating film precursor composed of a partial hydrolysis product. Such a coating film precursor dispersion liquid can be coated with good adhesion on the three-dimensional structure main body.

Alcohols such as methanol, ethanol, propanol, butanol, and ethylene glycol, ethers such as dimethyl ether, methyl tert-butyl ether, methyl propyl ether, diethyl ether, ethyl methyl ether, ethyl tert-butyl ether, and dibutyl ether, and the like are preferably used as the organic solvent.

The ratio of water used is preferably such that the amount of water is 0.1 parts by mole to 4 parts by mole and more preferably 0.5 parts by mole to 3 parts by mole per 1 part by mole of the alkoxide.

The ratio of the acid or alkali used is preferably such that the amount of acid or alkali is 0.01 parts by mole to 2.0 parts by mole and more preferably 0.05 parts by mole to 1.0 parts by mole, relative to 1 part by mole of the alkoxide.

In a preferable preparation example of the coating film precursor dispersion liquid, an alkoxide, water, an organic solvent and an acid are mixed in a molar ratio of alkoxide:water:organic solvent:acid = a:b:37:0.1 (provided that a is from 1.0 to 1.5, preferably 1, and b is from 1.0 to 1.5, preferably 1) to partially hydrolyze the alkoxide, and then dilution is preferably performed with the organic solvent to a predetermined ratio of the total amount of organic solvent used and the amount of alkoxide used.

The viscosity of the resulting coating film precursor dispersion liquid is preferably 0.8 mPa·s to 100 mPa·s as measured at 20°C.

### (4) Precursor Coating Step

In the precursor coating step, the coating film precursor dispersion liquid is coated on the surface of the three-dimensional structure main body to cover the surface of the three-dimensional structure main body with the coating film precursor.

The method for coating the coating film precursor dispersion liquid is not particularly limited. For example, a method of immersing the three-dimensional structure main body in the coating film precursor dispersion liquid and then separating (dip method), a method of spraying the coating film precursor dispersion liquid on the surface of the three-dimensional structure main body, a method of dropping the coating film precursor dispersion liquid on the surface of the three-dimensional structure main body, a method of coating the coating film precursor dispersion liquid on the surface of the three-dimensional structure main body with a brush or the like, a method of wetting the surface of the three-dimensional structure main body with the coating film precursor dispersion liquid, and the like can be used.

In particular, a method is preferred in which the three-dimensional structure main body is fixed to the below-described rotating plate, the three-dimensional structure main body is immersed in the coating film precursor dispersion liquid, and the three-dimensional structure main body is pulled up from the coating film precursor dispersion liquid and separated, thereby coating the coating film precursor on the surface of the three-dimensional structure main body. In such a coating method, the speed of pulling up the three-dimensional structure main body from the coating film precursor dispersion liquid is set, as appropriate, in consideration of the thickness of the coating film to be formed, the coating film precursor dispersion liquid, and the like, and is, for example, 1 mm/sec to 100 mm/sec.

### (5) Coating Film Forming Step

By rotating the three-dimensional structure main body coated with the coating film precursor in the sol state, it is possible to form a coating film made of an alkoxide partial hydrolysis product in the gel state or an alkoxide hydrolysis product obtained by a hydrolysis reaction and a polymerization reaction advanced by rotation, drying or the like.

As means for rotating the three-dimensional structure main body, a spin coater, a centrifuge, a centrifugal separator, and the like can be used.

Further, as a method of rotating the three-dimensional structure main body, it is preferable that the three-dimensional structure main body is fixed by fixing means such as screws to a rotating plate and the rotating plate is rotated to rotate the three-dimensional structure main body.

Rotation of the three-dimensional structure main body may be performed so that centrifugal force acts on the center of gravity of the three-dimensional structure main body. That is, the three-dimensional structure main body may be fixed to the rotating plate so that the center of gravity thereof is not positioned on the rotation axis of the rotating plate, but is preferably fixed so that the concave section and/or convex section in the three-dimensional structure main body is spaced apart from the rotation axis of the rotating plate, and more preferably fixed so that the whole of the three-dimensional structure main body is spaced apart from the rotation axis of the rotating plate.

The distance from the rotation axis (rotation axis of the rotating plate) to the center of gravity of the three-dimensional structure main body is set, as appropriate, according to the size of the rotating plate, but is preferably 5 mm or longer, and more preferably 10 mm to 1,000 mm.

Further, when the concave sections and/or convex sections of the three-dimensional structure main body are composed of grooves or ridges extending in one direction, the direction in which a centrifugal force acts may or may not be the same as the direction in which the grooves or ridges extend.

The relative centrifugal acceleration of the centrifugal force acting on the center of gravity of the three-dimensional structure main body is preferably 10 G to 500 G, more preferably 20 G to 250 G, and still more preferably 50 G to 200 G. When the relative centrifugal acceleration is within the above ranges, the surface of the three-dimensional structure main body can be coated with the coating film precursor with a more uniform thickness.

The centrifugal acceleration increases in proportion to the absolute value of the distance from the rotation axis and the square of the angular velocity of the rotational motion. That is, the centrifugal acceleration a is expressed by a = rω². Here, r is a position vector (radius) (m) from the center of rotation. Further, ω is an angular velocity (rad/s). The angular velocity is represented by ω = 2πN/60. Here, N is the revolution speed (rpm). The relative centrifugal acceleration RCF (G) is obtained from RCF (G) = a/g, where g is the earth gravity acceleration.

Therefore, the relative centrifugal acceleration of the centrifugal force acting on the center of gravity of the three-dimensional structure main body can be adjusted by changing either or both of the distance between the rotation axis and the center of gravity of the three-dimensional structure main body and the rotation speed related to the rotation of the three-dimensional structure main body.

Furthermore, the three-dimensional structure may be autorotated in the direction opposite to the rotation direction of the rotating plate while rotating the three-dimensional structure main body. By autorotating the three-dimensional structure main body while rotating, the centrifugal force acts on the three-dimensional structure main body from various directions, so that the three-dimensional structure main body can be coated with a more uniform thickness.

As the means for autorotating the three-dimensional structure main body, a rotary mechanism for autorotating each three-dimensional structure main body in the direction opposite to the rotation direction can be used. The rotary mechanism may be a small motor or may be a cam mechanism that reverses in the rotation direction. The rotational speed of the autorotating can be appropriately set, and is preferably 100 rpm to 10,000 rpm, and more preferably 1,000 rpm to 5,000 rpm.

Further, the coating film forming step can be performed simultaneously with the above-described precursor coating step. Specifically, the three-dimensional structure main body can be rotated while coating the coating film precursor dispersion liquid on the surface of the three-dimensional structure main body. However, in order to coat with a more uniform thickness, the coating film forming process is preferably performed after the precursor coating process, that is, the coating film precursor dispersion liquid is coated on the surface of the three-dimensional structure main body and the three-dimensional structure main body is then rotated. Further, where the three-dimensional structure main body is rotated after the coating film precursor dispersion liquid has been coated on the surface of the three-dimensional structure main body, it is preferable to start rotating the three-dimensional structure main body in a short time after the completion of the coating of the coating film precursor dispersion liquid on the surface of the three-dimensional structure main body. Specifically, the time from the completion of coating to the start of rotation is preferably within 5 min, and more preferably within 30 sec.

In the coating film forming step, by performing, as necessary, a drying of the coating film precursor in the sol state and/or the alkoxide hydrolysis product in the gel state, which has been coated on the three-dimensional structure main body, it is possible to form a coating film of the alkoxide hydrolysis product.

The drying may be natural drying in which the three-dimensional structure main body is stored indoors, or may be thermal drying in which the three-dimensional structure main body is heated.

In the case of performing thermal drying, the drying temperature is appropriately set according to the material of the three-dimensional structure main body, and is preferably 50°C to 200°C and more preferably 70°C to 140°C. When the material of the three-dimensional structure main body is a polymer, the temperature is set within a temperature range in which the polymer is not plasticized.

The drying time can be set as appropriate, and is suitably, for example, about 10 min to 48 h.

Further, where the material of the three-dimensional structure main body is a metal or ceramic, a firing treatment may be performed. The firing treatment temperature is suitably 200°C to 800°C. By performing the firing treatment, the crystallinity of the resulting coating film is increased, and functions such as bioactivity and catalytic activity for use in bone repair materials and the like may be exhibited or enhanced. The firing treatment time is suitably about 10 min to 48 h. The atmosphere for the firing treatment can be appropriately selected from an atmosphere in which oxygen is present, such as air, an inert gas atmosphere, a reducing gas atmosphere, and the like.

Since the coating film thus formed is formed of a metal alkoxide or non-metal alkoxide hydrolysis product, a coating film having a high light transmittance can be obtained. When the light transmittance of the coating film is expressed in terms of haze (cloudiness), it is preferably 5 or less, more preferably 3 or less, and most preferably 1 or less.

### (6) Bioactivation Treatment Step

When the three-dimensional structure of the present invention is used for a biomaterial such as a bone repair material, a bioactivation treatment is performed on the coating film. The bioactivation treatment is a treatment for forming apatite in vivo and expressing bone-bonding ability and is preferably an acid treatment disclosed in, for example, Patent Literature 1 and Non Patent Literature 1. The acid used in the acid treatment is preferably one or more aqueous solutions selected from hydrochloric acid, nitric acid, and sulfuric acid, and has a concentration of 0.001 mol/L to 5 mol/L. A particularly preferable concentration is 0.01 mol/L to 0.5 mol/L. When the acid treatment is performed within this range, the zeta potential of the coating can be charged to +3 mV to +20 mV in a relatively short time. After the acid treatment, washing and drying may be performed as appropriate. The drying temperature is such that the polymer is not plasticized, but a temperature of 50°C to 200°C is preferable and a temperature of 70°C to 140°C is more preferable. The drying time can be set as appropriate and is suitably, for example, about 10 min to 48 h.

According to the production method of the three-dimensional structure, a coating film having a uniform thickness and good adhesion can be formed even if a three-dimensional structure main body has a concave section and/or a convex section, and therefore, a three-dimensional structure having high durability can be produced without the coating film being peeled off even after long-term use.

### [Coating Device]

Fig. 2 is an explanatory diagram showing an outline of a configuration in an example of the coating device of the present invention. This coating device is for coating a metal alkoxide or non-metal alkoxide partial hydrolysis product or an alkoxide hydrolysis product obtained by a hydrolysis reaction and a polymerization reaction advanced by rotation, drying or the like on the surface of a three-dimensional structure main body 1 having a concave section and/or a convex section on the surface.

The coating device shown in Fig. 2 has a circular rotating plate 20 for fixing the three-dimensional structure main body 1, coating means 30 for coating a dispersion liquid including an alkoxide partial hydrolysis product on the surface of the three-dimensional structure main body 1, and rotating means 40 for rotating the three-dimensional structure main body 1 so that the centrifugal force acts on the center of gravity thereof.

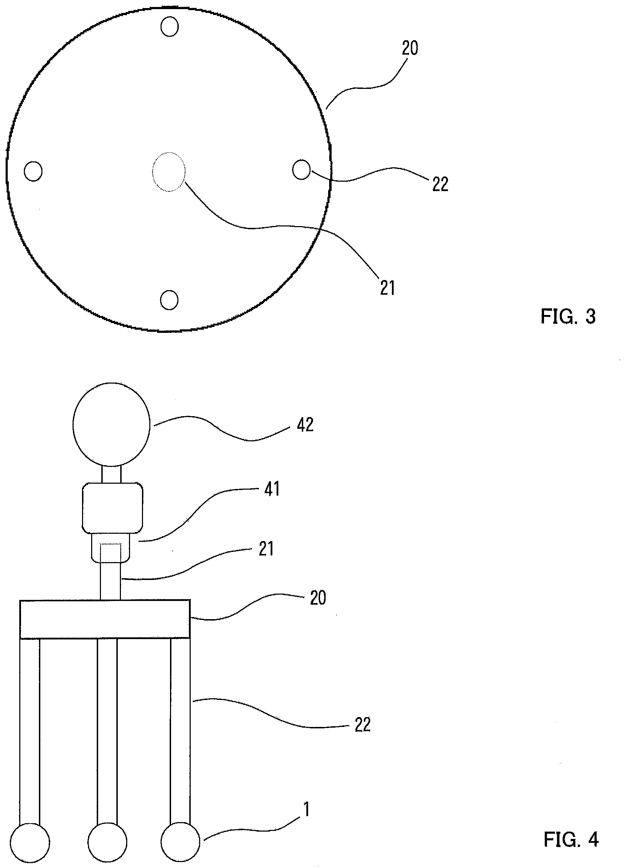

The rotating plate 20 is arranged horizontally. As shown in an enlarged view in Fig. 3, a rod 21 to be held by the rotating means 40 is provided at the center position on the upper surface of the rotating plate 20 so as to protrude upward. A plurality of (four in the illustrated example) rod-shaped fixtures 22 extending downward is arranged at the peripheral edge of the lower surface of the rotating plate 20 so as to be spaced apart from each other at equal intervals along the circumferential direction of the rotating plate 20. The three-dimensional structure main body 1 is fixed to the tip of each of the fixtures 22, and the three-dimensional structure main body 1 is thus fixed at a position set apart from the rotating plate 20 with the fixtures 22 being interposed therebetween.

An example of specific dimensions of the rotating plate 20 is as follows: the diameter of the rotating plate 20 is 100 mm; the rotating plate 2; the distance of 22 to the center axis is 10 mm).

The coating means 30 includes a dispersion liquid tank 31 filled with a coating film precursor dispersion liquid S, and an elevating mechanism 32 for raising and lowering the dispersion liquid tank 31. The elevating mechanism 32 is configured of a stage 33 on which the dispersion liquid tank 31 is disposed, a support column 35 that supports the stage 33 and extends upward from a base 34, and a stepping motor 36 that moves the stage 33 stepwise up and down in the extension direction of the support column 35.

As shown in Fig. 4, the rotating means 40 includes a holding member 41 that holds the rod 21 provided on the rotating plate 20, and a rotary motor 42 that rotates the rotating plate 20 via the holding member 41. The rotary motor 42 is held by the support column 35 in the coating means 30 through the support member 43.

In the coating device, first, a plurality of three-dimensional structure main bodies 1 is fixed to each of the fixtures 22 at the rotating plate 20. Next, when the stepping motor 36 of the elevating mechanism 32 is driven in the coating means 30 to raise the stage 33 on which the dispersion liquid tank 31 is disposed, each of the three-dimensional structure main bodies 1 is immersed in the film precursor dispersion liquid S in the dispersion liquid tank 31. Where the stage 33 is thereafter lowered by the stepping motor 36, each of the three-dimensional structure main bodies 1 is separated from the coating film precursor dispersion liquid S in the dispersion liquid tank 31. As a result, the coating film precursor dispersion liquid S is coated on each surface of the three-dimensional structure main body 1.

Where the rotating plate 20 is then rotated by the rotary motor 42 in the rotating means 40, each of the three-dimensional structure main bodies 1 is rotated. As a result, the centrifugal force acts on the center of gravity of each of the three-dimensional structure main bodies 1 (in the illustrated example, the entire three-dimensional structure main body 1). As a consequence, the surface of the three-dimensional structure main body 1 is coated with a uniform thickness with a metal alkoxide or non-metal alkoxide partial hydrolysis product of the coating film precursor, or an alkoxide hydrolysis product obtained by a hydrolysis reaction and a polymerization reaction advanced by rotation, drying or the like.

In the above process, the relative centrifugal acceleration of the centrifugal force acting on the center of gravity of the three-dimensional structure main body 1 is preferably 10 G to 500 G, more preferably 20 G to 250 G, and still more preferably 50 G to 200 G. Where the relative centrifugal acceleration is within the above ranges, the surface of the three-dimensional structure main body 1 can be coated with the coating film precursor with a more uniform thickness.

The distance from the rotation axis of the rotating plate 20 to the center of gravity of the three-dimensional structure main body 1 is set, as appropriate, according to the size of the rotating plate 20, but is preferably 5 mm or longer, and more preferably 10 mm to 1,000 mm.

With to the coating device, after the coating film precursor dispersion liquid S is applied to the surface of the three-dimensional structure main body 1 having a concave section and/or a convex sections by the coating means, the three-dimensional structure main body 1 is rotated by the rotating means so that the centrifugal force acts on the center of gravity thereof. Therefore, the surface of the three-dimensional structure main body can be coated with a coating film precursor with a uniform thickness in the range of, for example, 10 nm to 300 nm.

The coating device of the present invention is not limited to the above-described coating device, and the following various modifications can be added.
(1) In the coating device shown in Fig. 2, the surface of a plurality of three-dimensional structure bodies is coated with the coating film precursor, but the surface of only one three-dimensional structure main body may be coated with the coating film precursor.
(2) The rotating plate may have a structure in which the three-dimensional structure main body is directly fixed on the rotating plate. However, a structure in which the three-dimensional structure main body is directly fixed at a distance from the rotating plate, as shown in Fig. 2, is preferable.
(3) The rotating plate is not an essential configuration, and the three-dimensional structure main body may be fixed by other appropriate means.
(4) The coating means is not limited to that based on immersing the three-dimensional structure main body in the coating film precursor dispersion liquid, and the surface of the three-dimensional structure main body may be sprayed with the coating film precursor dispersion liquid, the coating film precursor dispersion liquid may be dropped on the surface of the three-dimensional structure main body, and the surface of the three-dimensional structure main body may be wetted with the coating film precursor dispersion liquid.
(5) The coating means may adopt a configuration in which the rotating plate on which the three-dimensional structure main body is fixed is moved up and down instead of the configuration in which the stage on which the dispersion liquid tank is arranged is moved up and down. Further, as means for separating the coating film precursor dispersion liquid from the three-dimensional structure main body, it is possible to use means in which the three-dimensional structure main body is disposed in a container having a through-hole formed in the peripheral wall, and the container is rotated, thereby removing the coating film precursor dispersion liquid through the through-hole of the container.
(6) A autorotating means for autorotating the three-dimensional structure main body while rotating the three-dimensional structure main body by the rotating means may be provided. For example, in the coating device shown in Fig. 2, the rotating plate 20 can be provided with autorotating means for autorotating the three-dimensional structure main body 1. The autorotating means is preferably one that autorotates in the direction opposite to the rotation direction of the rotating plate. By providing such autorotating means, the centrifugal force acts on the three-dimensional structure main body from various directions, so that the three-dimensional structure main body can be coated with a more uniform thickness.
(7) Drying means for drying the three-dimensional structure main body after the three-dimensional structure main body has been rotated by the rotating means may be provided. An electric heater, a steam heater, or the like can be used as the drying means. The heating temperature by the drying means is set, as appropriate, according to the material of the three-dimensional structure main body, and is, for example, 50°C to 200°C and preferably 70°C to 140°C. When the material of the three-dimensional structure main body is a polymer, the temperature is set within a temperature range in which the polymer is not plasticized.

### Examples

Hereinafter, the present invention will be described in greater detail with reference to specific examples and comparative examples. However, the present invention is not limited to these examples and can be implemented with arbitrary changes within a range not departing from the range of the claims of the present invention and the range of equivalents thereof.

### <Example 1>

### (1) Main Body Preparation Step

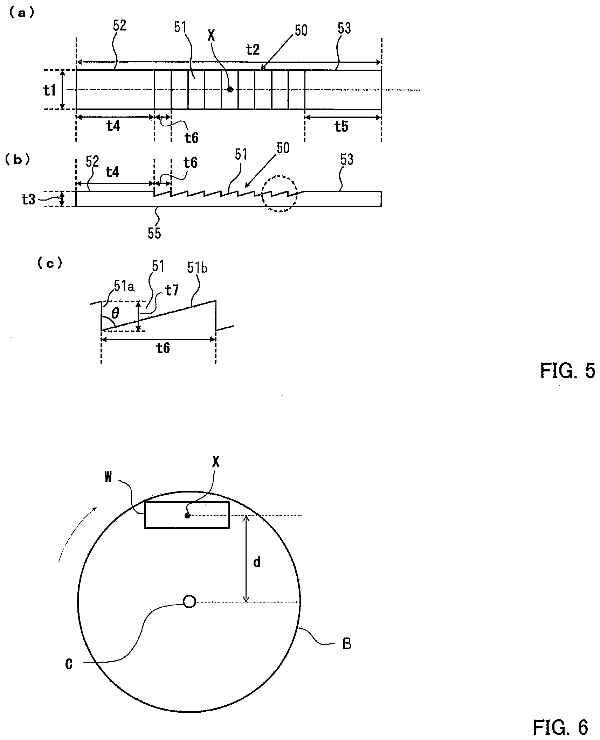

A three-dimensional structure main body (hereinafter referred to as "main body [A]") made of polyetheretherketone (PEEK) and having the form shown in Figs. 5(a)-(c) was prepared. The main body [A] is a rectangular plate having a vertical width (t1) of 5 mm, a horizontal width (t2) of 39.35 mm, and a thickness (t3) of 2 mm. The main body [A] has a concavo-convex section (50) in a central region in the long side direction on one surface thereof. The concavo-convex section portion (50) is formed such that nine wedge-shaped grooves (51) extending in the short side direction are arranged in the long side direction. The width (t6) of each groove (51) is 2.15 mm, and the depth (t7) of each groove (51) is 0.5 mm.

In the cross section of the main body [A] cut in the thickness direction along the long side direction, of the two sides related to the inner surface of the groove (51), one side (51a) extends in the thickness direction of the main body [A], the other side (51b) extends in a direction inclined in the thickness direction of the main body [A], and an angle θ formed by the one side (51a) and the other side (51b) is 76.9°.

Flat sections (52, 53) are respectively formed on both sides of the concavo-convex section (50) in the main body [A]. The widths (t4, t5) of the flat sections (52, 53) in the long side direction of the main body [A] are each 10 mm.

Further, the entire other surface of the main body [A] is a flat section (55).

The main body [A] was washed with ethanol, then washed with pure water, and thereafter dried with a dryer.

### (2) Main Body Modification Treatment Step

The surface of the main body [A] was subjected to modification treatment (hereinafter, this modification treatment is referred to as "O₂ plasma treatment") by using a vapor deposition device (IE-5) manufactured by Eiko Co., Ltd. under the conditions of an oxygen gas partial pressure of 10 Pa, plasma 0.6 kV-8 mA, an anode-main body [A] distance of 55 mm, and a treatment time of 5 min. The contact angle of water on the surface of the O₂ plasma-treated main body [A] was 20°.

### (3) Dispersion Liquid Preparation Step

A solution A was prepared by mixing 0.01 mol of titanium tetraisopropoxide (TTIP) and 0.185 mol of ethanol (EtOH). Further, a solution B was prepared by mixing 0.01 mol of water (H₂O), 0.185 mol of ethanol (EtOH), and 0.001 mol of nitric acid (HNO₃). A sol including a TTIP partial hydrolysis product was prepared by gradually adding the solution B dropwise while stirring the solution A. The molar ratio of components used in the preparation of the sol was TTIP : H₂O : EtOH : HNO₃ = 1 : 1 : 37 : 0.1. A coating film precursor dispersion liquid was prepared by diluting the sol with ethanol so that the molar ratio of the total amount of ethanol used and the amount of titanium tetraisopropoxide used was EtOH : TTIP = 37 : 0.8.

The hydrolysis rate of titanium tetraisopropoxide in the coating film precursor dispersion liquid was 25% in terms of mole.

The viscosity of the coating film precursor dispersion liquid was 2.5 mPa·s as measured at 20°C.

### (4) Precursor Coating Step

The main body [A] subjected to the O₂ plasma treatment was immersed in the coating film precursor dispersion liquid and submerged at a speed of 10 mm/sec, and the main body [A] was then pulled up at a speed of 10 mm/sec and separated to coat the surface of the main body [A] with the coating film precursor dispersion liquid.

### (5) Coating Film Forming Step

As shown in Fig. 6, the main body [A] (denoted by reference symbol W in Fig. 6) coated with the coating film precursor dispersion liquid was fixed on a rotating substrate B of a spin coater so that the distance d from the rotation axis C of the rotating substrate B to the center of gravity X of the main body [A] was 40 mm. Then, the rotating substrate B was rotated for 30 sec at a rotation speed of 1,500 rpm, thereby rotating the main body [A]. At this time, the relative centrifugal acceleration of the centrifugal force acting on the center of gravity X of the main body [A] was 100 G.

Next, the main body [A] was subjected to drying under conditions of 80°C for 24 hours to form a coating film on the surface of the main body [A], thereby producing a three-dimensional structure [A1].

### <Examples 2 to 5>

Three-dimensional structures [A2] to [A5] were produced in the same manner as in Example 1 except that the sol obtained was diluted with ethanol so that the amount of titanium tetraisopropoxide used relative to the total amount of ethanol used in the coating film precursor dispersion liquid preparation step was at ratios shown in Table 1.

### <Comparative Example 1>

A three-dimensional structure [B1] was produced in the same manner as in Example 1 except that the coating film precursor dispersion liquid was obtained without dissolving the sol with ethanol in the dispersion liquid preparation step, and that after the coating film precursor dispersion liquid was coated on the surface of the main body [A] in the precursor coating step, the main body [A] was immediately dried without rotating the main body [A] therebefore.

### <Comparative Examples 2 to 5>

Three-dimensional structures [B2] to [B5] were produced in the same manner as in Comparative Example 1 except that the sol obtained was diluted with ethanol so that the amount of titanium tetraisopropoxide used relative to the total amount of ethanol used in the coating film precursor dispersion liquid preparation step was at ratios shown in Table 1.

### [Evaluation]

The following evaluations (1) to (4) were performed with respect to the three-dimensional structures [A1] to [A5] obtained in Examples 1 to 5 and the three-dimensional structures [B1] to [B5] obtained in Comparative Examples 1 to 5.

### (1) Coating film thickness

The thickness of the coating film was measured by a method (calibration curve method) of measuring the optical density (UV absorptivity by regular transmitted light measured using "UV-2550" (manufactured by Shimadzu Corporation)) of a sample and calculating the film thickness by using a calibration curve prepared in advance. The results are shown in Table 1.

### (2) Adhesion of coating film

A transparent pressure-sensitive adhesive tape specified JIS K 5600 was affixed to the surface of the three-dimensional structure and peeled off. Here, "Cellotape (registered trademark)" manufactured by Nichiban Co., Ltd. and having a width of 25 mm and an adhesion strength of 4 N/10 mm was used as the transparent pressure-sensitive adhesive tape. The surface of the three-dimensional structure was visually observed, and evaluated as "O" when the coating film was not peeled off and "x" when the coating film was peeled off. The results are shown in Table 1.

### (3) Adhesion strength of coating film

The adhesion strength of the coating film in the three-dimensional structure was measured by a 180 degree peeling test in accordance with JIS K 6854. Specifically, a peeling test tape ("Y-4950" manufactured by 3M Co.) having a T-type peeling force (target SUS304) of 34 N/10 mm and a width of 10 mm was used, this peeling test tape was affixed to the surface of the coating film, the tape was pulled in the direction of 180° at a speed of 300 mm/min, the peel strength was measured, and the measured value was defined as the adhesion strength. The results are shown in Table 1.

### (4) Presence/absence of peeling of coating film

The surface of the flat sections (52, 53) and the concavo-convex section (50) in the three-dimensional structure was observed at a magnification of 300 and 1,000 by using a scanning electron microscope. Then, evaluation was made with "O" indicating that the coating film was not cracked or peeled and "×" indicating that the coating film was cracked or peeled. The results are shown in Table 1.

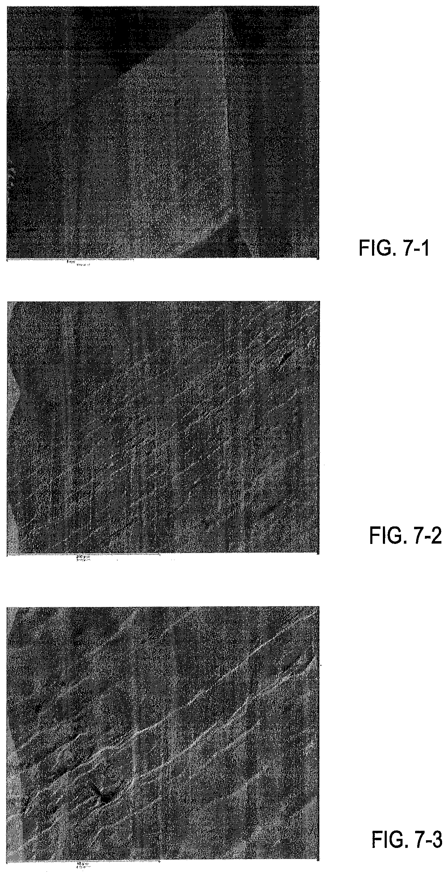

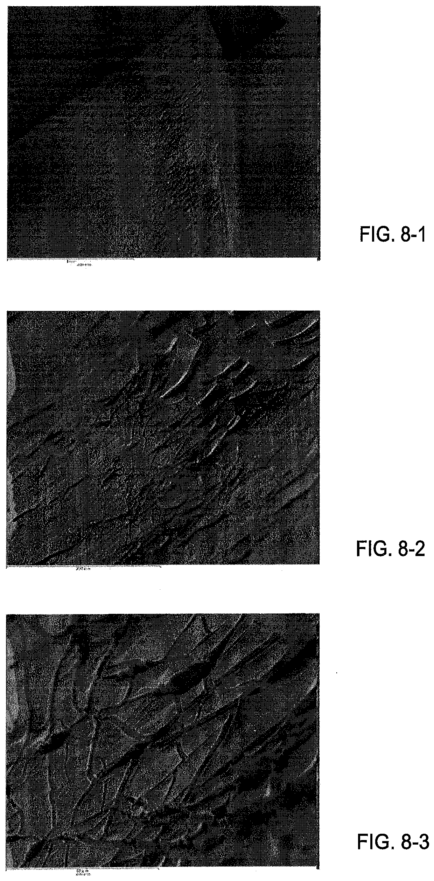

An electron micrograph taken at a magnification of 50 of the concavo-convex section in the three-dimensional structure obtained in Example 1 is shown in Fig. 7-1, an electron micrograph taken at a magnification of 300 is shown in Fig. 7-2, and an electron micrograph taken at a magnification of 1,000 is shown in Fig. 7-3. Further, an electron micrograph taken at a magnification of 50 of the concavo-convex section in the three-dimensional structure obtained in Comparative Example 1 is shown in Fig. 8-1, an electron micrograph taken at a magnification of 300 is shown in Fig. 8-2, and an electron micrograph taken at a magnification of 1,000 is shown in Fig. 8-3.

**[Table 1]**

| | | | Example 1 | Example 2 | Example 3 | Example 4 | Example 5 | Compar. Example 1 | Compar. Example 2 | Compar. Example 3 | Compar. Example 4 | Compar. Example 5 |
|---|---|---|---|---|---|---|---|---|---|---|---|---|
| Three-dimensional structure | | | A1 | A2 | A3 | A4 | A5 | B1 | B2 | B3 | B4 | B5 |
| TTIP : ethanol | | | 0.8 : 37 | 0.4 : 37 | 0.2 : 37 | 0.1 : 37 | 0.05 : 37 | 1 : 37 | 0.8 : 37 | 0.6 : 37 | 0.4 : 37 | 0.2 : 37 |
| Film thickness [nm] | | | 198 | 98 | 57 | 32 | 11 | 34 | 29 | 19 | 11 | 5 |
| Adhesion of coating film | | | ○ | ○ | ○ | ○ | ○ | ○ | ○ | ○ | ○ | ○ |
| Adhesion strength of coating film [N/10 mm] | | | 42 or higher | 42 or higher | 42 or higher | 42 or higher | 42 or higher | 42 or higher | 42 or higher | 42 or higher | 42 or higher | 42 or higher |
| Absence/ presence of peeling of coating film | 300x | Flat section | ○ | ○ | ○ | ○ | ○ | ○ | ○ | ○ | ○ | ○ |
| | | Concavo -convex section | ○ | ○ | ○ | ○ | ○ | × | × | × | × | ○ |
| | 1, 000 x | Flat section | ○ | ○ | ○ | ○ | ○ | ○ | ○ | ○ | ○ | ○ |
| | | Concavo -convex section | ○ | ○ | ○ | ○ | ○ | × | × | × | × | ○ |

Based on the results shown in Table 1, it was confirmed that the three-dimensional structures [A1] to [A5] according to Examples 1 to 5 have a coating film with good adhesion, without peeling of the coating film even in the concavo-convex section. Meanwhile, in the three-dimensional structures [B1] to [B5] according to Comparative Examples 1 to 4, peeling of the coating film was recognized at the concavo-convex section, and the adhesion of the coating film was low. Further, in the three-dimensional structure [B5] according to Comparative Example 5, peeling of the coating film is not recognized in the concavo-convex section, but the coating film is unlikely to be provided with a required function because the coating film is too thin.

### <Examples 6 to 10>

Three-dimensional structures [A6] to [A10] were produced in the same manner as in Example 1, except that the distance between the center of gravity X of the main body [A] and the rotation axis C of the rotating substrate B or the rotating speed of the rotating substrate B in the coating film forming step was changed according to Table 2 below. The obtained three-dimensional structures [A6] to [A10] were evaluated for the above-mentioned "(1) Thickness of coating film" and "(4) Presence/absence of peeling of coating film". The results are shown in Table 2 below.

**[Table 2]**

| | | | Example 6 | Example 7 | Example 8 | Example 9 | Example 10 |
|---|---|---|---|---|---|---|---|
| Three-dimensional structure | | | A6 | A7 | A8 | A9 | A10 |
| Rotation speed [rpm] | | | 500 | 1,100 | 1, 500 | 3, 000 | 1, 500 |
| Distance between center of gravity of main body and rotation axis [mm] | | | 40 | 40 | 40 | 40 | 10 |
| Relative centrifugal acceleration [G] | | | 11 | 54 | 101 | 403 | 25 |
| Film thickness [nm] | | | 53 | 47 | 45 | 41 | 45 |
| Absence/ presence of peeling of coating film | 300x | Flat section | ○ | ○ | ○ | ○ | ○ |
| | | Concavo-convex section | ○ | ○ | ○ | ○ | ○ |
| | 1, 000 x | Flat section | ○ | ○ | ○ | ○ | ○ |
| | | Concavo-convex section | ○ | ○ | ○ | ○ | ○ |

Based on the results of Table 2, it was recognized that in Examples 6 to 10, after the coating film precursor dispersion liquid was coated on the main body [A], the main body A was rotated so that the centrifugal force having a relative centrifugal acceleration in a specific range acted on the center of gravity of the main body A, whereby a coating film having good adhesion was obtained without peeling even in the concavo-convex section.

### [Industrial Applicability]

The three-dimensional structure of the present invention has various uses, for example, a catalyst, a catalyst support, an adsorbent, a photocatalyst, an electrode, an artificial bone, and the like depending on the material of the three-dimensional structure main body and the type of alkoxide hydrolysis product constituting the coating film.

### [Reference Sings List]

- 1: Three-dimensional structure main body
- 10: interbody cage
- 11: Through-hole
- 13: Ridge
- 12: Transverse hole
- 15: Side surface portion
- 16: Upper surface portion
- 17: Lower surface portion
- 20: Rotating plate
- 30: Coating means
- 40: Rotating means
- 21: Rod
- 22: Fixture
- 31: Dispersion liquid tank
- 32: Lifting mechanism
- 33: Stage
- 34: Table
- 35: Strut
- 36: Stepping motor
- 41: Holding member
- 42: Rotating motor
- 43: Support member
- 50: Concavo-convex section
- 51: Groove
- 51a: One side
- 51b: Other side
- 52, 53, 55: Flat portions
- B: Rotating substrate
- C: Rotation axis
- S: Coating film precursor dispersion liquid
- W: Main body [A]
- X: Center of gravity

## Claims

1. A three-dimensional structure comprising:
a three-dimensional structure main body having a concave section and/or a convex section on a surface, and having a coating film on the surface that includes the concave section and/or convex section of the three-dimensional structure main body, and the coating film has a thickness of 10 nm to 300 nm, wherein
the coating film includes a metal alkoxide or non-metal alkoxide hydrolysis product; and
no cracks or peelings of the coating film can be recognized when a portion of the coating film located on the surface of the concave section and/or convex section in the three-dimensional structure main body is observed with a scanning electron microscope at a magnification of 300.

2. The three-dimensional structure according to claim 1, wherein the coating film has an adhesion strength measured by a 180 degree peeling test in accordance with JIS K 6854 of 40 N/10 mm or higher.

3. The three-dimensional structure according to claim 1 or 2, wherein the three-dimensional structure main body is made of at least one material selected from the group consisting of polymers, metals, and ceramics.

4. The three-dimensional structure according to any one of claims 1 to 3, wherein the three-dimensional structure main body is made of polyetheretherketone.

5. The three-dimensional structure according to any one of claims 1 to 4, wherein the coating film includes a titanium alkoxide hydrolysis product.

6. A method for producing a three-dimensional structure comprising:
a main body preparation step of preparing a three-dimensional structure main body having a concave section and/or a convex section on a surface;
a dispersion liquid preparation step of preparing a coating film precursor dispersion liquid comprises a coating film precursor that includes an alkoxide partial hydrolysis product by mixing a metal alkoxide or non-metal alkoxide with water;
a precursor coating step of coating the coating film precursor dispersion liquid on the surface of the three-dimensional structure main body; and
a coating film forming step of forming a coating film that includes the alkoxide hydrolysis product on the surface of the three-dimensional structure main body by rotating the three-dimensional structure main body which has been coated with the coating film precursor dispersion liquid so that a centrifugal force acts on a center of gravity thereof.

7. The method for producing a three-dimensional structure according to claim 6, the method including a main body modification treatment step of modifying the surface of the three-dimensional structure main body before performing the precursor coating step.

8. The method for producing a three-dimensional structure according to claim 6 or 7, wherein a coating film includes an alkoxide hydrolysis product is formed by rotating the three-dimensional structure main body while coating the coating film precursor dispersion liquid on the surface of the three-dimensional structure main body.

9. The method for producing a three-dimensional structure according to claim 6, wherein
the three-dimensional structure main body is fixed to a rotating plate, and
the three-dimensional structure main body is immersed in the coating film precursor dispersion liquid and then separated from the coating film precursor dispersion liquid, thereby coating the coating film precursor dispersion liquid on the surface of the three-dimensional structure main body, and the three-dimensional structure main body is thereafter rotated by rotating the rotating plate to form a coating film includes the alkoxide hydrolysis product.

10. The method for producing a three-dimensional structure according to claim 9, wherein the three-dimensional structure main body is fixed so that the whole of the three-dimensional structure main body is spaced apart from the rotation axis of the rotating plate.

11. The method for producing a three-dimensional structure according to claim 9 or 10, wherein,
in the coating film forming step,
the three-dimensional structure main body is autorotated in the direction opposite to the rotation direction of the rotating plate while rotating the three-dimensional structure main body.

12. The method for producing a three-dimensional structure according to any one of claims 6 to 11, wherein,
in the dispersion liquid preparation step,
the coating precursor dispersion liquid is prepared by mixing 37 parts by mole of an organic solvent, 0.08 parts by mole to 1.5 parts by mole of a metal alkoxide or non-metal alkoxide, and water.

13. The method for producing a three-dimensional structure according to any one of claims 6 to 12, wherein the alkoxide is a titanium alkoxide.

14. The method for producing a three-dimensional structure according to any one of claims 6 to 13, wherein,
in the coating film forming step,
the relative centrifugal acceleration of the centrifugal force acting on the center of gravity of the three-dimensional structure main body is 10 G to 500 G.

15. The method for producing a three-dimensional structure according to any one of claims 6 to 14, wherein,
in the coating film forming step,
the three-dimensional structure main body is rotated and then subjected to drying at a temperature of 50°C to 200°C.

16. A coating device for coating a metal alkoxide or non-metal alkoxide partial hydrolysis product and/or alkoxide hydrolysis product on a surface of a three-dimensional structure main body having a concave section and/or a convex section on the surface, the coating device comprising:
coating means for coating a dispersion liquid including the alkoxide partial hydrolysis product on the surface of the three-dimensional structure main body; and
rotating means for rotating the three-dimensional structure main body so that a centrifugal force acts on a center of gravity thereof.

17. A coating device for coating a metal alkoxide or non-metal alkoxide partial hydrolysis product and/or alkoxide hydrolysis product on a surface of a three-dimensional structure main body having a concave section and/or a convex section on the surface, the coating device comprising:
a rotating plate for fixing the three-dimensional structure main body;
coating means for coating a dispersion liquid including the alkoxide partial hydrolysis product on the surface of the three-dimensional structure main body fixed to the rotating plate; and
rotating means for rotating the three-dimensional structure main body fixed to the rotating plate, so that a centrifugal force acts on a center of gravity of the three-dimensional structure main body.

18. The coating device according to claim 16 or 17, wherein the coating means is based on immersing the three-dimensional structure main body in the dispersion liquid.

19. The coating device according to claim 16 or 17, wherein the coating means is based on immersing the three-dimensional structure main body in the dispersion liquid and then separating the three-dimensional structure main body from the dispersion liquid.

20. The coating device according to claim 17, wherein the fixing position of the three-dimensional structure main body on the rotating plate is set apart from a rotation axis of the rotating plate.

21. The coating device according to claim 20, including autorotating means for autorotating the three-dimensional structure main body in the direction opposite to the rotation direction of the rotating plate.

22. The coating device according to claim 17, 20, or 21, wherein a plurality of three-dimensional structure main bodies, each constituting the three-dimensional structure main body, is fixed to the rotating plate.

23. The coating device according to any one of claims 16 to 22, wherein the rotating means applies a centrifugal force having a centrifugal acceleration of 10 G to 500 G to a center of gravity of the three-dimensional structure main body.

24. The coating device according to any one of claims 16 to 23, including drying means for drying the alkoxide partial hydrolysis product and/or alkoxide hydrolysis product coated on the surface of the three-dimensional structure main body.
